# EUROPEAN PATENT APPLICATION

(11) **EP 1 804 188 A1**
(43) Date of publication of application: **04.07.2007**
(21) Application number: 06127313.2
(22) Date of filing: 28.12.2006
(51) Int. Cl.: G06F 19/00

(54) **Method for conditioning body weight and device for implementing this method.**

(30) Priority: 30.12.2005 IT BO20050803
(71) Applicant: Technogym S.p.A., 47035 Gambettola (Forli Cesena) (IT)
(72) Inventor: Roman, Maurizio, 30033 Noale (VENEZIA) (IT)
(74) Representative: Lanzoni, Luciano

(57) **Abstract**

A device (1) for conditioning the weight of a user comprises: measuring means (2) for measuring at least one physical characteristic of the user and coding it in a weight signal (7); a computerised control unit (8) in communication with the measuring means (2) for processing the weight signal (7); a display (9) in communication with the computerised unit (8) for displaying a plurality of indications preferably defined by the computerised unit (8); the device (1) also comprising means (23) for calculating a quantity of energy which can be used by the user to condition the relevant physical characteristic.

## Description

The present invention relates to a device and a method for processing information concerning the achievement and maintenance of personal fitness, in particular for conditioning weight.

The present invention lies, in particular, in the field of improved scales.

Many types of scales are known which as well as performing the normal function of measuring body weight, are integrated with a plurality of devices designed to provide the generic user with a plurality of items of information linked to the physical state and metabolism of the user.

For example, there are prior art scales able to estimate the number of calories to be consumed in order to maintain or reach a predetermined body weight.

Other types of scales have devices designed to calculate the energy consumption necessary to maintain or reach a predetermined weight based on the basal metabolism.

There are also prior art scales which save and display, using a suitably connected external interface, for example a personal computer, body weight fluctuations in predetermined time intervals or variations in body composition (lean mass, fat mass, percentage of liquids, etc.) over time.

Prior art types of scales have several disadvantages.

In particular, prior art scales do not provide the user with a valid aid for planning a lifestyle which can help to achieve an optimum physical condition.

In particular, devices for conditioning body weight, that is to say scales, of the conventional type provide a daily total of calories to be taken in or used up based on estimated data affected by an intrinsic error.

For example, for calculating the calories used up by a person reference is often made to the basal metabolism. That method, and therefore the devices which operate using it, is affected by an error which accumulates day by day without the possibility of correcting the indications of calories to be taken in/used up supplied to the user because the energy consumption due to the basal metabolism is substantially approximate.

For example, an error in excess in estimating the basal metabolism results in overestimation of the daily calorie intake the user is allowed, which, over a predetermined period, will cause an accumulation of excess calories and a weight increase without the device noticing the error.

In this context, the main technical purpose of the present invention is to propose an improved device for conditioning body weight which is free of the above-mentioned disadvantages.

The present invention has for an aim to propose a method and device for conditioning body weight which are not affected by intrinsic errors due to the use of approximate estimated data.

The present invention also has for an aim to propose a device and method which are a valid aid to the generic user to optimise his lifestyle.

The present invention has for a further aim to propose a device which can provide the user with differentiated suggestions for substantially controlling his own body weight.

Further features and advantages of the present invention are more apparent in the detailed description below, with reference to a preferred, non-limiting embodiment of scales, illustrated in the accompanying drawings, in which:
- Figure 1 is an assembly drawing, in functional blocks, of a device in accordance with the present invention;
- Figure 2 is a block diagram showing, in greater detail, several characteristic components of the invention of Figure 1, in a second embodiment;
- Figure 3 is a schematic example diagram of a preferred method of operation of the device in accordance with the present invention.

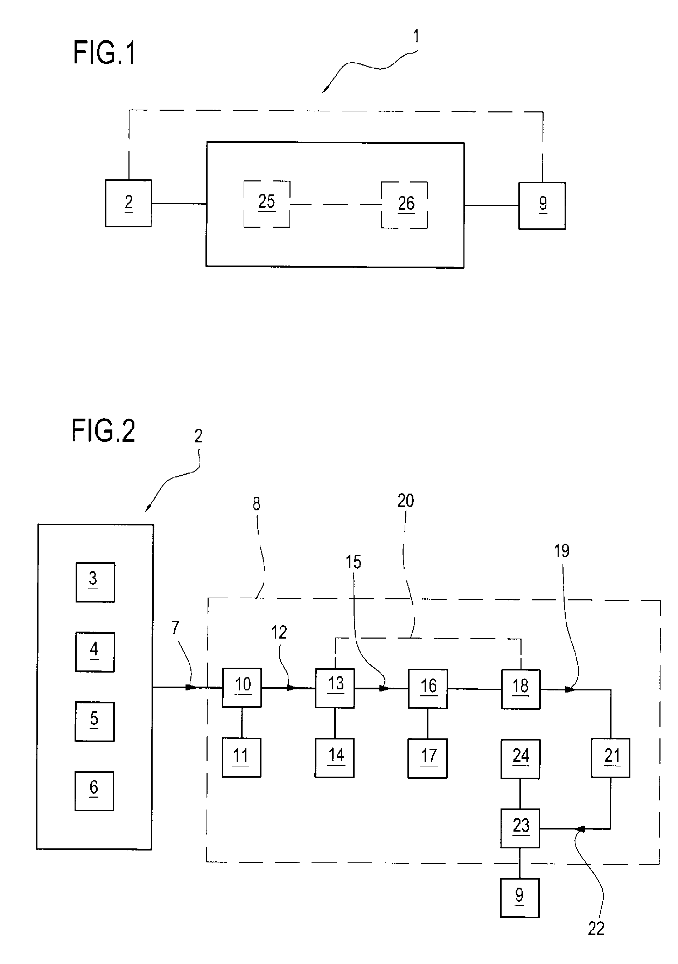

With reference to the accompanying drawings and in particular with reference to Figure 1, the numeral 1 denotes a device for conditioning body weight in accordance with the present invention.

It should be noticed that the device 1 described below is designed to condition a plurality of physical characteristics of the generic user. The device 1 is preferably intended to condition the body weight of the user, in particular since he is able to configure the device 1 as scales.

The device 1 comprises measuring means for measuring a plurality of physical characteristics of a generic user, not illustrated, the measuring means schematically illustrated with a block 2.

As illustrated in Figure 2, the means 2 for measuring a plurality of physical characteristics preferably comprise means for measuring the body weight of the user, of the substantially known type, schematically illustrated with a block 3.

The means 2 for measuring a plurality of physical characteristics preferably comprise means for measuring the fat mass of the user, of the substantially known type, schematically illustrated with a block 4.

The fat mass is preferably defined as a percentage of the weight of the user.

The means 2 for measuring a plurality of physical characteristics preferably comprise means for measuring the lean mass of the user, of the substantially known type, schematically illustrated with a block 5.

The lean mass is preferably defined as a percentage of the weight of the user.

The means 2 for measuring a plurality of physical characteristics preferably comprise means for measuring the percentage of liquids in the body of the user, of the substantially known type, schematically illustrated with a block 6.

The measuring means 2 supply at output a signal 7, for the sake of simplicity in this description called the weight signal, preferably digital, generated depending on the physical characteristics of the user measured by the means 2.

The weight signal 7 is supplied at input to a computerised control unit 8 described in more detail below.

A display 9 is associated with the unit 8 to supply the user with a plurality of indications, including body weight or any of the other physical characteristics measured.

Advantageously, in alternative embodiments not illustrated, the display 9 is substituted by any other means for viewing or communication with the user.

Said indications also preferably comprise indications obtained or calculated by the unit 8 depending on the weight signal 7.

As illustrated with a dashed line in Figure 1, the display 9 may be in direct communication with the measuring means 2 to display said characteristics without the computerised unit 8 inserted between them.

As illustrated in Figure 2, the unit 8 comprises a plurality of electronic components, of the substantially known type, in communication with one another to define the above-mentioned indications for the user.

A first calculation or conditioning centre, schematically illustrated with a block 10 receives at input the weight signal 7 and cleans it up, in the substantially known way, removing any interference factors.

More precisely, the first calculation centre 10 is in communication with a first memory, or first memory cell, schematically illustrated with a block 11, in which a plurality of data relative to the weight of the user is saved.

In this first memory a plurality of data linked to the interference factors is also sampled or saved.

The data relative to the weight of the user comprises an average of the weights measured in a previous predetermined time interval.

Said average is preferably a moving average.

The moving average is preferably an exponential moving average.

Advantageously, the data relative to the weight of the user can be saved in any suitable form.

The above-mentioned data relative to interference factors preferably comprises information relative to user water retention and/or user fat mass.

Advantageously, the information relative to user water retention is interpolated with the percentage of liquids measured in the body by the means 6 for measuring the percentage of liquids.

The data relative to the interference factors is preferably relative to user clothing sampling.

The data relative to the interference factors is preferably relative to the time of the day, for example the hour, when the weight and other physical characteristics are measured.

Advantageously, the first memory 11 can save data and information about any preferred interference factor which may intervene in the measurement of the weight of the user.

Therefore, it should be noticed that at output from the first calculation centre 10, the computerised control unit 8 has a signal 12, preferably depending on the weight, fat mass and lean mass, cleaned of so-called "noise" due to interference factors which are substantially attenuated and reduced.

A second calculation centre, schematically illustrated with a block 13 receives at input the noise-free signal 12.

Said calculation centre 13 is in communication with a second memory, or second memory cell, schematically illustrated with a block 14, in which a plurality of data relative to the weight of the user is saved or sampled.

Saved in the memory 14 there is preferably a plurality of user body weight values, in particular free of the above-mentioned noise, and calculated during previous measurements.

The calculation centre 13, interpolating the noise-free signal 12 with the data saved, defines a trend relative to the future value of the user body weight.

In practice, the body weight trend over time is interpolated.

The estimated trend is preferably linear. Advantageously, in other embodiments, the estimated trend is exponential.

In particular, the block 13 defines an estimated weight value 100, at a final moment t_{f} , where the term moment preferably refers to a day selected as the end of a period of observation.

At output from the block 13, the unit 8 has a signal 15 relative to the future value of the user body weight.

A third calculation centre, schematically illustrated with a block 16, receives the signal 15 at input to identify a suitable target weight for the user.

In greater detail, the calculation centre 16 in is in communication with a third memory, or third memory cell, schematically illustrated with a block 17, in which a plurality of user physiological characteristics is saved or sampled.

The memory 17 preferably contains data such as the user height, waist measurement, gender, age and type of physical activity, together with any other appropriate data item relative to the user.

Said data is preferably integrated with characteristics such as the fat mass, preferably obtained from the measurements taken by the measuring means 2.

The calculation centre 16, interpolating the signal 15 with the physiological characteristics, defines a user weight value, for the sake of simplicity called the target weight 101, optimum for the psychophysical well-being of the user, preferably to be reached in a predetermined time, that is to say, by the final moment t_{f}.

Using the above-mentioned methods, the calculation centre 16 preferably defines a plurality of intermediate user weight values between the current weight and the target weight, that is to say, it defines a plurality of intermediate stages in a path for reaching the target weight at the final moment t_{f}.

In practice, the centre 16 defines a suitable and advantageous user weight trend over time which probably differs, particularly at the final moment, from the trend defined in the block 13 and by the target weight 101.

It is important to notice that the device 1 calculates the target weight 100 if it does not already have a target weight set or calculated previously.

In practice, the target weight preferably remains constant until the final moment t_{f} is reached.

A fourth calculation centre, schematically illustrated with a block 18, calculates the difference between the target weight value 101 and the final weight value 100, calculated, that is to say expected, when defining the trend.

The block 18 therefore supplies at output a difference signal 19 supporting the information relative to the difference between the target weight and the estimated weight, otherwise known as the deviation, in particular at the final moment t_{f}.

It should be noticed that the calculation centre 18 is also designed to calculate differences between intermediate target values and corresponding intermediate values calculated in the trend at corresponding times; for a simple description reference is made to the difference between the weights, target and estimated 101, 100, at the final moment t_{f}.

The block 18 therefore receives at input the target weight value 101 and the weight value 100 estimated from the trend, the latter preferably from the block 16 or directly from the second calculation centre 13 in communication with the calculation centre 18 by means of a line 20 illustrated with a dashed line, and supplies the difference signal 19 at output.

The signal 19 is supplied at input to means 21 for calculating the energy the user must take in, or use up, in order to reach the target weight.

The quantity of energy defined is preferably divided by the means 21 by fractions of time, that is to say, the quantity of energy to be taken in or used up for example in a day or in a week or in any preferred time interval in order to reach the target weight is calculated.

The means 21 for the energy calculation generate a signal 22 which is an expression of a delta (Δ) of energy substantially corresponding to how much more or less energy the user must take in or use up compared with a previous reference period.

In other words, the delta of energy is calculated depending on the actual energy used up by the user weighted with the plurality of body weight values saved in the block 14 and with the user physiological characteristics saved in the block 17.

It should be noticed that the signal 22 which is an expression of a delta of energy to be taken in or used up substantially depends on the actual energy used up by the user, since it depends on the previous weighings and is not therefore affected by errors due to experimental estimates of the energy used up, for example the basal metabolism.

The previous weighings are an expression, in particular, of the basal metabolism, of physical activity and of the organic material expelled.

More precisely and by way of example, with t₀ the moment the device 1 is used, the quantity of energy defined by the calculation means 21 is divided by the number of days separating the moment of use t₀ from the final moment t_{f}.

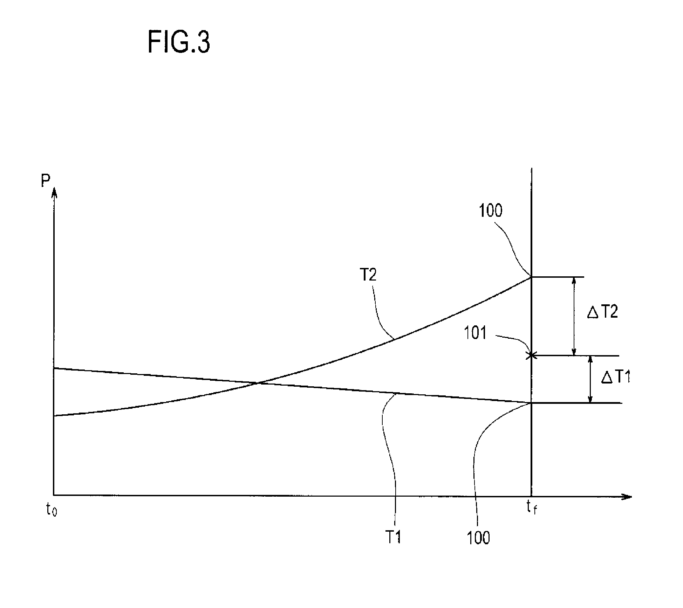

In particular, Figure 3 shows an example graph of device 1 interpolations where t₀ and t_{f} have the meanings indicated, 101 is the target weight, 100 the estimated user weight at the moment t_{f}, whilst T1 and T2 are two different example trends, the first exponential and the second linear, estimated by the calculation centre 13, where ΔT1 and ΔT2 are the deviation between the target weight 101 and the estimated weight 100 in the two trends considered by way of example.

It should be noticed that the final weight value 100 estimated in the trend T1, T2 may be greater than or less than the target weight.

In the case of trend T1 the weight value 100 estimated at the moment t_{f} is less than the target weight 101 and therefore, since the corresponding ΔT1 is positive, the user must take in a positive delta of energy to bridge the gap, that is to say, he must take in more energy than that usually taken in until the moment t₀.

Vice versa, in the case of trend T2 the weight value 100 estimated at the moment t_{f} is greater than the target weight 101 and therefore, since the corresponding ΔT2 is negative, the user must use up energy, that is to say lose weight, to reduce the corresponding gap; in particular in the case of a negative ΔT2 he must use up more energy than that usually used up until the moment t₀.

Said quantity of energy is preferably calculated in calories.

When the delta of energy which the user must take in is calculated, the means 21 preferably multiply the difference signal 19 by a suitable conversion factor.

This conversion factor preferably corresponds, as has been observed in experiments, to 7000 cal/kg.

The signal 22 is input into a fifth calculation centre, schematically illustrated with a block 23, which, depending on the signal 22, interacts with a fifth memory cell, schematically illustrated with a block 24.

In particular, catalogued in the memory cell 24 there is a plurality of activities, preferably physical or food, with predetermined energy usage levels.

Depending on the delta calculated for how much more or less energy the user must take in or use up compared with his habits to bridge the gap between the target weight and the weight estimated in the trend, the block 23 interrogating the memory 24, identifies the optimum activities for reaching the target weight, that it so say, the optimum activities for supplying the user with the delta of energy or taking it away from the user.

Such activities preferably comprise physical exercises and foods which should or should not be eaten.

The activities identified and selected by the block 23 are then shown on the display 9.

As illustrated in Figure 1, advantageously, the memories 11, 14, 17, 24 are configured in a single component schematically illustrated with a block 25 shown with a dashed line and suitably interrogated by the relative calculation centre.

Similarly, the calculation centres 10, 13, 16, 18, 23 and the means 21 for calculating the energy are integrated in a single processor, commonly known as a chip, schematically illustrated with a block 26 shown with a dashed line.

The device 1 is preferably designed to define and supply to the user, preferably by means of the display 9, an indication of the weight of the user expected with the elapsing of a predetermined time interval if the food intake and physical activity of the user do not change relative to those of a predetermined time interval, for example a week, prior to the moment t₀ the measurements were taken. The present invention also relates to a method for conditioning the body weight of the generic user.

The method comprises the step of measuring at least one quantity relative to the physical state of the user.

The weight of the user is preferably periodically measured.

The weight measurement may advantageously be integrated with measurements of the lean mass, the fat mass, the percentage of liquids in the body, the type of physical activity, the waist measurement and the gender of the user.

The values obtained are cleaned of any interference, due for example to water retention, the type of clothing or the time of day in which the measurement is taken.

Then a user weight trend is calculated substantially depending on the weights measured and "cleaned up".

In particular said trend has a final value, that is to say, corresponding to a generic final moment t_{f} when the weight conditioning will be ended.

Depending on the trend obtained and the physiological characteristics of the user, an optimum target weight is defined, which will promote the good health of the user.

It is also preferably possible to estimate intermediate weights between the weight measurement moment considered and the final moment, to set a path for reaching the target weight.

Therefore, there are two weight trends, one estimated based on the measurements and one calculated preferably based on a desired final target weight.

It is therefore possible to calculate, moment by moment, or day by day rather than from one week to the next, a deviation of the actual weight from the corresponding target weight at the moment considered.

This deviation is translated into a delta of energy to be taken in or used up by the user compared with a previous period of observation, that is to say, relative to the quantity of energy normally taken in, to substantially make the two items coincide at the final moment, that is to say, to reach the target weight.

As indicated, said energy is preferably in the form of calories which the user must take in or use up.

Depending on said delta of energy, the user is supplied with a weighted list of other physical activities, in addition to those performed until the moment t₀ of measurement, to carry out, or a list of foods which he must avoid or eat corresponding to the delta of calories to be used up or taken in.

The invention described brings important advantages.

It accurately supplies a delta of energy to be taken in or used up compared with the energy taken in or used up in a period prior to the measurement in order to reach the weight and fat mass targets appropriately defined.

It allows the weight (and many other parameter such as the fat mass, lean mass and percentage of water) to be monitored and controlled and allows their sampling for easy interpolation and consultation even by the user.

The device helps the user to reach a target weight as an indicator of healthy physical fitness, therefore supplying indications regarding the lifestyle of the user.

The invention described has evident industrial applications and may be modified and adapted in several ways without thereby departing from the scope of the inventive concept. Moreover, all details of the invention may be substituted by technically equivalent elements.

## Claims

1. A device for conditioning the weight of a user comprising measuring means (2) for measuring at least one physical characteristic of the user and coding it in a weight signal (7), a computerised control unit (8) in communication with the measuring means (2) for processing the weight signal (7), display means (9) in communication with the computerised unit (8) for displaying a plurality of indications preferably defined by the computerised unit (8), the device being **characterised in that** it comprises means (23) for calculating a quantity of energy which can be used by the user to condition said physical characteristic, the physical characteristic preferably being the body weight.

2. The device according to claim 1, **characterised in that** the quantity of energy which can be used is expressed as how much more or less energy can be taken in or used up compared with a quantity of energy usually taken in by the user in a predetermined time interval.

3. The device according to claim 2, **characterised in that** the computerised unit (8) comprises calculation means (26) operating in conjunction with storage means (25) to define a trend relative to the future value of the physical characteristic, the calculation means (26) which operate in conjunction with the storage means (25) comprising the means (23) for calculating a quantity of energy.

4. The device according to claim 3, **characterised in that** the calculation means (26) operate in conjunction with the storage means (25) to identify a target value for the physical characteristic.

5. The device according to claim 4, **characterised in that** the calculation means (26) are designed to assign to the target value, in particular to the target weight, a preferred final moment (t_{f}) for reaching the target weight.

6. The device according to claim 5, **characterised in that** the calculation means (26) are designed to define a plurality of intermediate values of the physical characteristic, between the value of the physical characteristic at a generic moment (t₀) of device use and the target value.

7. The device according to claim 5 or 6, **characterised in that** the calculation means (26) are designed to calculate, at each moment, the deviation between the physical characteristic target value and the value of the physical characteristic measured, in particular at the final moment (t_{f}).

8. The device according to claim 7, **characterised in that** the deviation is processed by the means (23) for calculating a quantity of energy.

9. The device according to claim 8, **characterised in that** the means (23) for calculating a quantity of energy are designed to convert the deviation into a quantity of energy using a conversion factor, said quantity of energy being an expression of how much more or less energy must be taken in or used up compared with the quantity of energy usually taken in by the user in the predetermined time interval.

10. The device according to claim 9, **characterised in that** the calculation means (26) are designed to multiply the deviation by the conversion factor.

11. The device according to claim 10, **characterised in that** the conversion factor is 7000 cal/kg, the quantity of energy preferably being expressed in calories.

12. The device according to any of the claims from 3 to 11, **characterised in that** the calculation means (26) operate in conjunction with the storage means (25) to identify optimum activities so that the physical characteristic reaches the target value, there being catalogued in the storage means (25) a plurality of activities, preferably physical or food, with predetermined energy usage levels.

13. The device according to claim 12, **characterised in that** the calculation means (26) are in communication with the display means (9) for displaying the optimum activities for reaching the target value.

14. The device according to any of the claims from 1 to 13, **characterised in that** the measuring means (2) comprise means (3) for measuring the body weight of the user.

15. The device according to any of the claims from 1 to 14, **characterised in that** the measuring means (2) comprise means (4) for measuring the fat mass of the user.

16. The device according to any of the claims from 1 to 15, **characterised in that** the measuring means (2) comprise means (5) for measuring the lean mass of the user.

17. The device according to any of the claims from 1 to 16, **characterised in that** the measuring means (2) comprise means (6) for measuring the percentage of liquids in the body of the user.

18. The device according to any of the claims from 1 to 17, **characterised in that** the control unit (8) comprises means (10) for conditioning the weight signal (7) to eliminate weight signal (7) interference factors.

19. The device according to claim 18, **characterised in that** the weight signal (7) conditioning means (10) operate in conjunction with means (11) for storing data relative to the weight of the user.

20. The device according to claim 19, **characterised in that** the storage means (11) sample data linked to the interference factors.

21. The device according to claim 3, **characterised in that** the storage means (25) contain a plurality of user body weight values preferably free of the interference factors.

22. The device according to claim 3, **characterised in that** the trend is linear.

23. The device according to claim 3, **characterised in that** the trend is exponential.

24. The device according to any of the claims from 1 to 23, **characterised in that** the storage means (25) save a plurality of user physiological characteristics.

25. The device according to claim 24, **characterised in that** the physiological characteristics comprise the age of the user.

26. The device according to claim 24 or 25, **characterised in that** the physiological characteristics comprise the height of the user.

27. The device according to any of the claims from 24 to 26, **characterised in that** the physiological characteristics comprise the gender of the user.

28. The device according to any of the claims from 24 to 27, **characterised in that** the physiological characteristics comprise the waist measurement of the user.

29. The device according to any of the claims from 24 to 28, **characterised in that** said physiological characteristics are integrated with physiological characteristics measured by the measuring means (2) .

30. A method for conditioning the body weight of a person comprising the steps of measuring at least one physical characteristic of the person, in particular the weight, and coding it in a weight signal, **characterised in that** it comprises the step of estimating a trend for the physical characteristic over time, defining at least one target value, at a suitable moment, for the physical characteristic, calculating the deviation of said target from the corresponding estimated value at said moment, translating said deviation into at least one activity to be performed in order to reduce the deviation.

31. The method according to claim 27, **characterised in that** it comprises a step of calculating a quantity of energy which is how much more or less to be taken in or used up compared with a quantity of energy usually taken in by the user in a predetermined time interval, said activity to be performed preferably being defined depending on the quantity of energy which is how much more or less to be taken in or used up compared with the quantity of energy usually taken in by the user in said predetermined time interval.

32. The method according to claim 30 or 31,
**characterised in that** it comprises the step of defining intermediate target values of the physical characteristic to set a path for reaching the target value.

33. The method according to any of the claims from 30 to 32, **characterised in that** it comprises the step of integrating the measurement of the physical characteristic, preferably the weight, with measurements of the lean mass or the fat mass present in the person's body.

34. The method according to any of the claims from 30 to 33, **characterised in that** it comprises the step of integrating the measurement of the physical characteristic, preferably the weight, with a measurement of the percentage of liquids in the person's body.

35. The method according to any of the claims from 30 to 34, **characterised in that** it comprises the step of filtering the weight signal to remove any interference.

36. The method according to any of the claims from 30 to 35, **characterised in that** it comprises the step of translating the deviation into an energy variation, in particular calories.

37. The method according to any of the claims from 30 to 35, **characterised in that** it comprises the step of supplying the person with a weighted list of said activities depending on the energy variation.
